# EUROPÄISCHE PATENTANMELDUNG

(11) **EP 1 952 692 A1**
(43) Veröffentlichungstag der Anmeldung: **06.08.2008**
(21) Anmeldenummer: 07019229.9
(22) Anmeldetag: 29.09.2007
(51) Int. Cl.: A01N 59/16, A01N 59/20, A61K 33/08, A61K 33/30, A61K 33/34, A61K 33/38, C01B 39/02

(54) **Verfahren zur Herstellung eines antibakteriell bzw. antimikrobiell wirkenden keramsichen Werstoffes sowie dessen Verwendung**

(30) Priorität: 10.01.2007 DE 102007001466
(71) Anmelder: S & B Industrial Minerals GmbH, 45772 Marl (DE)
(72) Erfinder: Genske, Dr. Dieter, 45657 Recklinghausen (DE); Kesore, Dr. Kisnaduth, 45772 Marl (DE); Tzintzos, Spyridon, 15234 Halandri, Athen (GR)
(74) Vertreter: Nunnenkamp, Jörg

(57) **Zusammenfassung**

Gegenstand der vorliegenden Erfindung ist ein Verfahren zur Herstellung eines antibakteriell bzw. antimikrobiell wirkenden keramischen Werkstoffes auf Basis eines porösen keramischen Trägermaterials mit an- oder eingelagerten Metallionen auf Silber- und/oder Kupfer- und/oder Zinkbasis. Erfindungsgemäß werden die Metallionen per Festkörper-lonenaustausch in trockener Phase mit dem Trägermaterial verbunden.

## Beschreibung

Die Erfindung betrifft ein Verfahren zur Herstellung eines antibakteriell bzw. antimikrobiell wirkenden keramischen Werkstoffes auf Basis eines porösen keramischen Trägermaterials mit an- und/oder eingelagerten Metallionen auf Silber- und/oder Kupfer- und/oder Zinkbasis.

Ein derartiges Verfahren wird beispielhaft in der DE 40 36 298 C2 beschrieben. Hier geht es um einen antibakteriell wirksamen keramischen Werkstoff, bei welchem die fraglichen Metallionen in wässriger Phase als wässriges Metallsalz von keramischem Trägermaterial adsorbiert werden. Hierbei kann es sich um Zeolith handeln.

Darüber hinaus sind Körperpflegemittel bekannt, die metallische Partikel wie beispielsweise Silber und Zink enthalten. Sobald das fragliche Körperpflegemittel in Kontakt mit Körperflüssigkeit oder Körperfeuchtigkeit kommt, werden Zinkionen und Silberionen freigesetzt, die die gewünschte antibakterielle bzw. antimikrobielle Wirkung sicherstellen (vgl. DE 103 40 276 B4).

Schließlich beschreibt die WO 00/06208 A1 eine Zahnpasta, welche antimikrobielle Keramikpartikel bzw. Zeolith enthält. In dem Zeolith ist ein Teil der austauschbaren Ionen durch antimikrobiell wirksame Silber- und Zinkionen ersetzt worden.

Die antimikrobielle bzw. antibakterielle Wirkung von Silber- und/oder Kupfer- und/oder Zinkionen ist grundsätzlich bekannt. So haben bereits die Griechen und Römer Silbermünzen zur Wasserdesinfizierung genutzt. Selbst die NASA greift im Space Shuttle auf Silber zurück, um die Wasserreinheit zu gewährleisten. Der Effekt beruht vermutlich darauf, dass Silberionen die Energieproduktion durch die Blockade entsprechender Enzyme der Bakterien verhindern, welche folgerichtig absterben, und zwar ohne negative Effekte auf beispielsweise menschliche Zellen.

Zinkionen wird in Verbindung mit Silberionen eine gegenseitige Unterstützung in ihrer antimikrobiellen Wirkung nachgesagt, wobei Zink zusätzlich entzündungshemmend wirkt. - Kupfersalze bzw. Kupferionen werden bei der Weinherstellung eingesetzt, um die Schimmel- und Pilzbildung sowie Schlammbildung zu unterdrücken. In diesem Zusammenhang wird die toxische Wirkung von Kupfer ausgenutzt.

Der Stand der Technik nach der DE 40 36 298 C2 hat sich grundsätzlich bewährt. Allerdings erfolgt die Einlagerung der Metallionen in das poröse keramische Trägermaterial auf nasschemischem Wege, so dass etwaige flüssige und ggf. toxische sowie schwer zu entsorgende flüssige Rückstände entstehen. Hier setzt die Erfindung ein.

Der Erfindung liegt das technische Problem zugrunde, ein derartiges Verfahren so weiter zu entwickeln, dass bei verringerten Kosten eine umweltschonende Herstellung gelingt.

Zur Lösung dieser technischen Problemstellung ist ein gattungsgemäßes Verfahren dadurch gekennzeichnet, dass die Metallionen per Festkörper-lonenaustausch in trockener Phase mit dem Trägermaterial verbunden werden.

Ein solcher Festkörper-lonenaustausch ist grundsätzlich bekannt, wozu auf die WO 2006/094720 A1 verwiesen sei, allerdings in anderem Zusammenhang. Ähnliches gilt für den Aufsatz von M. Crocker et al. "Preparation of acidic forms of montmorillonite clay via solid-state ion-exchange reactions" CATALYSIS LETTERS, Bd. 15, 1992, Seiten 339-345. Beim Festkörper-lonenaustausch worden die an- bzw. einzulagernden Metallionen vorteilhaft als Metallsalze, vorzugsweise Metallnitratsalze, oder Metallsulfatsalze trocken mit dem Trägermaterial gemischt. Tatsächlich empfiehlt die Erfindung den Rückgriff auf beispielsweise Silbernitrat (AgNO₃), Kupfernitrat (Cu(NO₃)₂) und Zinksulfat (ZnSO₄).

Zuvor können die einzelnen Mischungskomponenten gemahlen werden. Alternativ oder zusätzlich ist auch ein nachgeschalteter Mahlvorgang denkbar. Dabei wird üblicherweise mit einer Mühle mit Mahlkörpern gearbeitet. Das heißt, die Mischung respektive die Mischungskomponenten werden in eine Mahltrommel mit regelmäßig kugelförmigen Mahlkörpern verbracht, die schlagend die gewünschte Mahlfeinheit zur Verfügung stellen. Dabei haben sich für den keramischen Werkstoff vor und/oder nach dem Festkörper-lonenaustausch Korngrößen bis zu 500 µm, insbesondere maximal 250 µm, als besonders günstig erwiesen. Die minimale Korngröße liegt in der Regel bei 2 µm, höchstens 5 µm. Dabei ist es denkbar, den Mahlvorgang mit dem Heizvorgang für den Festkörper-lonenaustausch zu kombinieren. Hierfür kann die Mahltrommel beheizt werden. Alternativ oder zusätzlich ist es möglich, mit erhitzten Mahlkörpern zu arbeiten.

In Folge der solchermaßen erreichten Mahlfeinheit ist der keramische Werkstoff für eine Vielzahl anschließender Verwendungen prädestiniert. Beispielsweise kann das poröse keramische Trägermaterial mit den an- oder eingelagerten Metallionen als Zusatz für Körperpflegemittel, Reinigungsmittel oder auch als Kunststoffzusatz Verwendung finden. In letztgenanntem Fall wird der Kunststoff primär in Verbindung mit Lebensmitteln eingesetzt bzw. muss geschmacksneutral respektive lebensmittelecht ausgeführt werden. Denkbare Anwendungen sind hier Aufbewahrungsbehälter für Lebensmittel aber auch Spielzeuge für Babys und Kleinkinder.

Im Rahmen des Festkörper-lonenaustausches wird die Mischung aus dem porösen keramischen Trägermaterial und dem Metallsalz bzw. den Metallsalzen in der Regel auf Temperaturen bis zu 600° C, vorzugsweise bis zu 500° C erhitzt. Dabei wird meistens eine schockartige Temperaturzunahme verfolgt, wobei die Erfindung in der Regel mit einem Gradienten von ca. 10° C/min für die Temperaturerhöhung arbeitet. Auch größere Gradienten sind denkbar. Immer ist zu gewährleisten, dass der Festkörper-lonenaustausch bei der Trockenmischung aus dem Trägermaterial mit dem Metallsalz bei Temperaturen von mehr als 300° C stattfindet.

Die maximale Temperatur mag bei ca. 500° C bzw. 600° C liegen. Jedenfalls sorgt die schockartige Erhitzung dafür, dass die Kationen in den überwiegend eingesetzten Gerüstsilikaten bzw. Tektosilikaten teilweise durch die Metall-kationen, also Silberionen und/oder Kupferionen und/oder Zinkionen ersetzt werden. Das geschieht in der Trockenphase. Durch das beschriebene Erhitzen der Trockenmischung kommt es gleichzeitig oder in einem nachgeschalteten Vorgang dazu, dass der keramische Werkstoff kalziniert wird. Damit ist gemeint, dass in der Trockenmischung evtl. vorhandenes Kristallwasser oder andere Lösungsmittel entfernt werden und gleichzeitig Kohlendioxid eine Abspaltung erfährt. Das Kalzinieren kann nach dem Festkörper-lonenaustausch erfolgen und für wenigstens eine Stunde durchgeführt werden. Vorzugsweise wird eine Zeitdauer von bis zu drei Stunden verfolgt, um die Verteilung der Metallionen innerhalb des üblicherweise eingesetzten Gerüstsilikates zu vergleichmäßigen. Gleichzeitig wird durch diesen Vorgang der behandelte keramische Werkstoff insgesamt stabilisiert. Als Temperatur für den Vorgang des Kalzinierens empfiehlt die Erfindung Werte von 100° C bis 200° C.

Neben den Metall-Kationen, die sich üblicherweise im Innern des porösen keramischen Trägermaterials durch lonenaustausch anlagern, sammeln sich Cluster oder Aggregate der Metallionen in oxidischer oder auch metallischer (reiner) Form auf der äußeren Oberfläche des fraglichen Trägermaterials an, werden also an das betreffende Trägermaterial angelagert. Das hängt wesentlich von der Behandlung, insbesondere der Temperaturbehandlung ab. Dabei gilt der Grundsatz, dass die äußere Anlagerung umso stärker bzw. ausgeprägter ist, je höher die Temperatur für den Festkörper-lonenaustausch und auch der Gradient gewählt wurden.

Der Gehalt an Metallionen in dem solchermaßen behandelten porösen keramischen Trägermaterial beläuft sich auf ca. 1 bis 10.000 ppm. Die spezifische und für die An- bzw. Einlagerung der Metallionen relevante Oberfläche des porösen keramischen Trägermaterials liegt im Bereich von ca. 25 m²/g bis 300 m²/g. Die Dichte des Trägermaterials mag im Bereich von in etwa 2 bis 2,5 g/cm³ liegen.

Da als Trägermaterial in der Regel Alumosilikate und hier insbesondere Tektosilikate bzw. Gerüstsilikate im Allgemeinen und Zeolithe im Speziellen Verwendung finden, wird ein Verhältnis Silizium/Aluminium von in etwa 4 bis 20 beobachtet. Die Kationenaustauschkapazität des porösen keramischen Trägermaterials mag im Bereich von mehr als 15 meq/100 g, vorzugsweise mehr als 30 meq/100 g, angesiedelt sein.

Bei dem eingesetzten Trägermaterial bzw. Gerüstsilikat handelt es sich überwiegend um ein natürliches Mineral, insbesondere natürlichen Zeolith. Dabei können neben Klinoptilolith grundsätzlich Chabasit, Mortinit usw. oder Mischungen hiervon zum Einsatz kommen. Tatsächlich hat es sich bewährt, wenn das eingesetzte Gerüstsilikat wenigstens 50 Gew.-% an natürlichem Zeolith enthält, wobei der Rest beispielsweise von künstlichen Zeolithen gebildet werden kann. Das eingesetzte natürliche Zeolith enthält mehr als 50 Gew.-%, vorzugsweise mehr als 70 Gew.-% Klinoptilolith. Bekanntermaßen handelt es sich bei Klinoptilolith um ein Aluminiumsilikat, welches anionisch reagiert und aufgrund seiner Gitterstruktur sowie der hohen inneren und äußeren Oberfläche und Porosität als lonenaustauscher gegenüber den lediglich als Kationen ausgebildeten Metallionen auf Silber- und/oder Kupfer- oder Zinkbasis besonders prädestiniert ist.

## Patentansprüche

1. Verfahren zur Herstellung eines antibakteriell bzw. antimikrobiell wirkenden keramischen Werkstoffes auf Basis eines porösen keramischen Trägermaterials mit an- und/oder eingelagerten Metallionen auf Silber- und/oder Kupfer- und/oder Zinkbasis, **dadurch gekennzeichnet, dass** die Metallionen per Festkörper-lonenaustausch in trockener Phase mit dem Trägermaterial verbunden werden.

2. Verfahren nach Anspruch 1, **dadurch gekennzeichnet, dass** der keramische Werkstoff vor und/oder nach dem Festkörper-lonenaustausch gemahlen wird, wobei Korngrößen bis zu 500 µm, insbesondere maximal 250 µm, eingestellt werden.

3. Verfahren nach Anspruch 1 oder 2, **dadurch gekennzeichnet, dass** der keramische Werkstoff kalziniert wird.

4. Verfahren nach Anspruch 3, **dadurch gekennzeichnet, dass** das Kalzinieren mehr als eine Stunde, vorzugsweise bis zu drei Stunden, erfolgt.

5. Verfahren nach einem der Ansprüche 1 bis 4, **dadurch gekennzeichnet, dass** der Gehalt an Metallionen in dem Trägermaterial bis zu 10.000 ppm beträgt.

6. Verfahren nach einem der Ansprüche 1 bis 5, **dadurch gekennzeichnet, dass** der Festkörper-lonenaustausch bei einer Trockenmischung aus dem Trägermaterial mit einem Metallsalz bei Temperaturen von mehr als 300° C stattfindet.

7. Verfahren nach einem der Ansprüche 1 bis 6, **dadurch gekennzeichnet, dass** die Temperatur für den Festkörper-lonenaustausch schockartig mit einem Gradienten von ca. 10° C/min oder mehr erhöht wird.

8. Verfahren nach einem der Ansprüche 1 bis 7, **dadurch gekennzeichnet, dass** die maximale Temperatur bei dem Festkörper-lonenaustausch ca. 600° C beträgt.

9. Verfahren nach einem der Ansprüche 1 bis 8, **dadurch gekennzeichnet, dass** als Trägermaterial ein Alumosilikat, insbesondere ein Gerüstsilikat, vorzugsweise Zeolith, eingesetzt wird.

10. Verwendung eines nach dem Verfahren gemäß der Ansprüche 1 bis 9 hergestellten antibakteriell bzw. antimikrobiell wirkenden keramischen Werkstoffes als Zusatz für Körperpflegemittel, Reinigungsmittel oder als Kunststoffzusatz.
